# EUROPÄISCHE PATENTSCHRIFT

(11) **EP 1 451 151 B1**
(45) Veröffentlichungstag und Bekanntmachung des Hinweises auf die Patenterteilung: **14.01.2009**
(21) Anmeldenummer: 02791683.2
(22) Anmeldetag: 15.11.2002
(51) Int. Cl.: C07C 323/52, A61K 31/192, A61P 7/02, C12R 1/645

(54) **HYDROPERYLEN-DERIVATE**
HYDROPERYLENE DERIVATIVES
DERIVES D'HYDROPERYLENE

(30) Priorität: 28.11.2001 DE 10158402
(43) Veröffentlichungstag der Anmeldung: 01.09.2004
(73) Patentinhaber: Sanofi-Aventis Deutschland GmbH, 65929 Frankfurt am Main (DE)
(72) Erfinder: KURZ, Michael, 65719 Hofheim (DE); HERRMANN, Matthias, 65719 Hofheim-Diedenbergen (DE); TOTI, Luigi, 65239 Hochheim (DE); VERTESY, Laszlo, 65817 Eppstein-Vockenhausen (DE)
(86) Internationale Anmeldenummer: PCT/EP2002/012802
(87) Internationale Veröffentlichungsnummer: WO 2003/045905

(56) Entgegenhaltungen:
- ARNONE, A ET AL: "Secondary mould metabolites. Part 16. Stemphyltoxins, new reduced perylenequinone metabiolites from Stemphylium botryosum var. Lactucum" JOURNAL OF THE CHEMICAL SOCIETY, PERKIN TRANSACTIONS 1., 1986, Seiten 525-530, XP002235228 CHEMICAL SOCIETY. LETCHWORTH., GB ISSN: 0300-922X in der Anmeldung erwähnt
- ROBESON, D ET AL: "Alteichin: an unusual phytotoxin from Alternaria eichorniae, a fungal pathogen of water hyacinth" EXPERIENTIA., Bd. 40, 1984, Seiten 1248-1250, XP002235229 BIRKHAUSER VERLAG. BASEL., CH ISSN: 0014-4754 in der Anmeldung erwähnt
- OKUNO, T ET AL: "Structure of Antifungal and Phytotoxic Pigments produced by alternaria sps." TETRAHEDRON LETTERS., Bd. 24, Nr. 50, 1983, Seiten 5653-5656, XP002235230 ELSEVIER SCIENCE PUBLISHERS, AMSTERDAM., NL ISSN: 0040-4039 in der Anmeldung erwähnt
- STIERLE, A ET AL : "Phytotoxins from Alternaria Alternata, a pathogen of spotted knapweed" JOURNAL OF NATURAL PRODUCTS., Bd. 52, Nr. 1, 1989, Seiten 42-47, XP001148771 XX, XX ISSN: 0163-3864 in der Anmeldung erwähnt
- KROHN, K ET AL : "Biologically active metabolites from fungi 13. Stemphytriol, a new perylene derivative from monodictys fluctuata" NATURAL PRODUCT LETTERS., Bd. 14, Nr. 1, - 1999 Seiten 31-34, XP008014826 HARWOOD ACADEMIC PUBLISHERS, CHUR., CH ISSN: 1057-5634

## Beschreibung

Die Erfindung betrifft Hydroperylen-Derivate, die von dem Pilz ST003367, DSM 14452, während der Fermentation gebildet werden oder nachträglich derivatisiert werden, Verfahren zu deren Herstellung und die Verwendung derselben als Arzneimittel und Inhibitoren der Blutplättchen.

Hydroperylen-Derivate, wie Hexahydro- oder Octahydro-perylen-Derivate werden von verschiedenen Mikroorganismen gebildet. Bekannte Hydroperylen-Derivate sind beispielweise:
- Altertoxine I, II und III (M. E. Stark et al. J. Nat. Prod. 49, 866-871, 1986), das
- Alteichin (D. Robeson et al. Experientia, 40, 1248-1250, 1984), die
- Alterlosine (A. Stierle et al. J. Nat. Prod.. 52, 42-47, 1989) und das
- Alterperylenol aus Alternaria Spezies (T. Okuno et al. Tetrahedron Lett. 24, 5653-5656, 1983);
- Stemphyltoxine sowie das Stemphypyrenol, die aus Kulturen des Stemphylium botryosum (A. Arnone et al. J. Chem. Soc. Perkin Trans.1, 1986, 525-530) stammen.

Die Altertoxine und die Stemphyltoxine sind Phytotoxine, die durch ihre mutagene Wirkung auch den Wert der menschlichen Lebensmittel beeinträchtigen können. Sie sind deshalb in über 70 Publikationen gut beschrieben worden (T. J. Schrader et al. Teratog., Carcinog., Mutagen. 21, 261-274, 2001). Naturgemäß werden hierbei die Gefahren, die von den Altertoxinen und Stemphyltoxinen ausgehen, behandelt; ein Ergebnis der Untersuchungen ist die weite Verbreitung und die krebserregende Wirkung dieser Epoxide (Oxirane) enthaltenden Verbindungen. Darüber hinaus ist über eine schwache Telomerase-inhibierende Wirkung des Alterperylenols berichtet worden (K.-I. Togashi et al. Oncol. Res. 10, 449-453, 1998), der für die Enzymhemmung charakteristische IC₅₀-Wert ist jedoch mit 30 µM gering.

Thromboembolische Erkrankungen sind - insbesondere in den westlichen Industrienationen - die häufigste Todesursache. Einer wirksamen Prophylaxe und Therapie dieser Erkrankung kommt somit eine außerordentliche Bedeutung zu. Unter einem Thrombus versteht man ein intravital und intravasal entstandenes Blutgerinnsel. Thromben entstehen nach Thrombozytenaggregation vor allem in Arterien. Begünstigt wird die Thrombenbildung durch Gefäßwandschädigung, verlangsamte Blutströmung und beschleunigte Gerinnung.

Die Thrombozyten (Blutplättchen) sind diskusförmige, kernlose Blutzellen, die im Verletzungsfall die Blutstillung und Blutgerinnung gewährleisten. Die Thrombozyten bewirken die Blutstillung in einem komplizierten Vorgang durch Aggregation, die Thrombozytenaggregation ist somit ein für Warmblüter lebenswichtiger Vorgang. Die Unterfunktion der Thrombozyten führt schon bei kleineren Verletzungen zu schweren Blutungen, andererseits begünstigt eine erhöhte Gerinnungsneigung die Thrombose-und Emboliegefahr. Da insbesondere die Plättchen-Überfunktion häufig fatale Folgen hat, wurde bereits mehrere Substanzen als Thrombozytenaggregations-Hemmstoffe eingesetzt. Bekannt ist die Acetylsalicylsäure (Aspirin®), das Ticlopidin (Tiklyd®) oder das verwandte Clopidogrel, aber all diese Präparate sind aufgrund von Nebenwirkungen nur begrenzt einsetzbar. Deshalb besteht ein großer Bedarf an Inhibitoren der intrazellulären Plättchenaktivierung für die Therapie und Langzeit-Prophylaxe von arteriellen thrombo-embolischen Ereignissen. Eingesetzt werden können solche Mittel beispielsweise beim Myocardinfarkt, bei der instabilen Angina oder bei Schlaganfällen.

In dem Bestreben, wirksame Verbindungen zur Vorbeugung oder Behandlung von Blutgerinnungs-Erkrankungen zu finden, wurde nun gefunden worden, dass Thioperylenol, der vom Pilzstamm ST 003367, DSM 14452, gebildet wird und weitere Hydroperylen-Derivate die Blutplättchen-Aggregation wirksam hemmen können.

Die Erfindung betrifft daher die Verbindung der Formel I 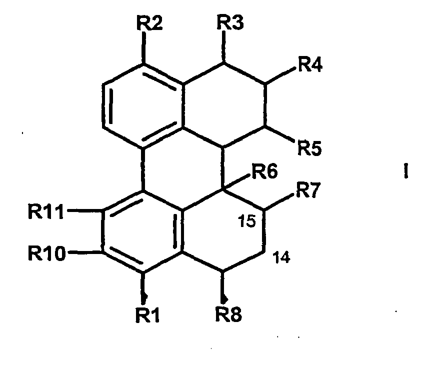 und/oder alle stereoisomere Formen der Verbindung der Formel I und/oder
Gemische diese Formen in jedem Verhältnis, und/oder ein physiologisch verträgliches Salz der Verbindung der Formel I, wobei
R1, R3 und R8 unabhängig voneinander für -OH oder =O stehen,
R2 für -OH steht,
R4 und R5 unabhängig voneinander für -OH oder Wasserstoffatom stehen, oder R4 und R5 zusammen mit den Kohlenstoffatomen an die sie jeweils gebunden sind ein Epoxid bilden,
R6, R10 und R11 unabhängig voneinander für -OH oder Wasserstoffatom stehen, oder
R10 und R11 zusammen mit den Kohlenstoffatomen an die sie jeweils gebunden sind ein Epoxid bilden,
R7 für den Rest -S-CH₂-CHOH-COOH steht,
und die Bindung zwischen -C₁₄-C₁₅- eine Einfachbindung oder eine Doppelbindung ist.

Die Erfindung betrifft auch eine Verbindung der Formel I, wobei
R1, R2, R3 und R6 jeweils für -OH stehen,
R4 und R5 zusammen mit den Kohlenstoffatomen an die sie jeweils gebunden sind ein Epoxid bilden,
R7 für den Rest -S-CH₂-CHOH-COOH steht,
R8 für =O steht,
R10 und R11 jeweils für Wasserstoffatom stehen und
die Bindung zwischen -C₁₄-C₁₅- eine Einfachbindung ist,
und/oder physiologisch verträgliche Salze der Verbindung der Formel I.

Die Erfindung betrifft ferner die Verbindung der Formel II

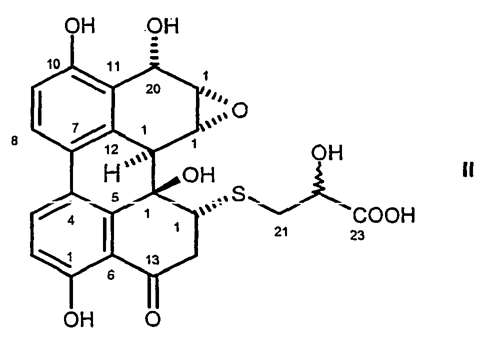

Die Verbindung der Formel II wird im folgenden äls Thioperylenol bezeichnet (Summelformel: C₂₃H₂₀NO₉S; Molekulargewicht 472,47).
sowie deren physiologisch verträglichen Salze.

Die Erfindung betrifft ferner ein Verfahren zur Herstellung der Verbindung der Formel I, und/oder einer stereoisomeren Form der Verbindung der Formel I und/oder Gemische diese Formen in jedem Verhältnis, und/oder ein physiologisch verträgliches Salz der Verbindung der Formel I, das dadurch gekennzeichnet ist, dass man
a) den Mikroorganismus DSM 14452 oder dessen Mutanten oder Varianten in einem wäßrigen Nährmedium kultiviert und die Verbindung Thioperylenol isoliert und reinigt oder
b) Thioperylenol durch chemische Derivatisierung in eine Verbindung der Formel I überführt, oder
c) eine nach den Verfahren a) oder b) hergestellte Verbindung der Formel I, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder
d) die nach den Verfahren a), b) oder c) hergestellte Verbindung der Formel I entweder in freier Form isoliert oder im Falle des Vorliegens von sauren oder basischen Gruppen in physiologisch verträgliche Salze umwandelt.

Der Mikroorganismus DSM 14452 (interne Bezeichnung ST003367) gehört zur Gruppe der Pilze und besitzt ein weisses Substratmycel und ganz wenig Luftmycel und wurde am 3. August 2001 unter den Bedingungen des Budapester Vertrags bei der DSMZ-Deutsche Sammlung von Mikroorganismen und Zellkulturen GmbH, Mascheroder Weg 1 b, D-38124 Braunschweig unter der Nummer DSM 14452 hinterlegt.

Unter Varianten von DSM 14452 werden Stämme von DSM 14452 verstanden, die durch Vereinzelung aus einer Kultur von DSM 14452 gewonnen wurden, soweit sie Thioperylenol herstellen. Unter Mutanten von DSM 14452 werden Stämme von DSM 14452 verstanden, die nach Mutation aus einer Kultur von DSM 14452 gewonnen wurden, soweit sie Thioperylenol herstellen. Mutanten von DSM 14452 können in an sich bekannter Weise durch physikalische Mittel, beispielsweise Bestrahlung wie Ultraviolett- oder Röntgenstrahlung oder durch chemische Mutagene, beispielsweise Ethylmethansulfonat (EMS); 2-Hydroxy-4-methoxy-benzophenon (MOB) oder N-Methyl-N'-nitro-N-nitrosoguanidin (MNNG) erzeugt werden.

Das Auffinden der Mutanten erfolgt beispielsweise durch Probennahme aus dem Kultivierungsmediums und Bestimmung der inhibierenden Wirkung von Thioperylenol.

Die Herstellung von Thioperylenol erfolgt durch Kultivierung von DSM 14452. Die Nährlösung enthält Kohlenstoffquellen wie Saccharose, Maisstärke, Dextrose, Lactose, D-Mannit, Molasse oder Malzextrakt und Stickstoffquellen wie Sojabohnenmehl, Erdnußmehl, Proteine, Peptone, Peptide, Tryptone, Fleischextrakt, Hefeextrakt oder Ammoniumsalze oder Nitrate.

Die Nährlösung enthält auch anorganische Salze wie Natriumhydrogenphosphat, Natriumchlorid, Calciumchlorid, Calciumsulfat, Calciumcarbonat, Magnesiumsulfat oder Kaliumhydrogenphosphat. Ferner kann dem Nährmedium auch Fett wie Ölsäuremethylester oder Sojaöl zugesetzt werden. Daneben werden auch Spurenelemente wie Eisen-, Mangan-, Kupfer-, Zink-, Kobalt- oder andere Metallsalze zugegeben.

Eine bevorzugte Nährlösung enthält etwa von 0,05 % bis 5 % Kartoffeldextrose, vorzugsweise von 1 % bis 3 %, und von etwa 0,05 % bis 3 % Hefeextrakt, vorzugsweise von 0,05 % bis 1 %. Die Prozentangaben beziehen sich auf das Gewicht der gesamten Nährlösung.

Die Kultivierung von DSM 14452 erfolgt bei Temperaturen von 18 °C bis 35 °C, bevorzugt bei 23 °C bis 28 °C und bei pH-Werten von 3 bis 10, bevorzugt 5 bis 9, vorzugsweise bei Werten von 4 bis 6. Die Kultivierung erfolgt aerob zunächst im Schüttelkolben und danach im Fermenter unter Rühren und Belüftung mit Luft oder reinem Sauerstoff. Die Kultivierung der Mikroorganismen in den Fermentern erfolgt über einen Zeitraum von 48 bis 720 Stunden, bevorzugt von 72 bis 144 Stunden.

Die Bildung von Thioperylenol erreicht ihr Maximum nach etwa 96 bis 144 Stunden. In der Nährlösung bildet der Pilzstamm DSM 14452 auch Gemische von mehreren Verbindungen der Formel I. Je nach Zusammensetzung der Nährlösung kann der mengenmäßige Anteil eines oder mehrerer der Verbindungen der Formel I variieren. Außerdem kann durch die Medienzusammensetzung die Synthese einzelner Verbindungen der Formel I gesteuert werden, so dass einzelne Verbindungen der Formel I gar nicht oder in einer Menge unterhalb der Nachweisgrenze vom Mikroorganismus hergestellt werden.

Die Isolierung von Thioperylenol erfolgt direkt aus der Nährlösung oder nach Abtrennung der Zellen beispielsweise durch Zentrifugation oder Filtration. Die Isolierung von Thioperylenol kann durch Extraktion mit Lösungsmitteln oder durch Adsorption an Harze wie XAD 16, HP 20, MCI Gel® CHP20P oder Ionenaustauscher erfolgen.
Die Reinigung erfolgt beispielsweise durch Chromatographie an Adsoptionsharzen wie an Diaion^{®} HP-20 (Mitsubishi Casei Corp., Tokyo), an Amberlite^{®} XAD 7 (Rohm and Haas, USA), an Amberchrom^{®} CG (Toso Haas, Philadelphia, USA). Die Trennungen können in einem weiten pH-Bereich durchgeführt werden. Bevorzugt ist der Bereich von pH 1 bis pH 9, besonders bevorzugt von pH 2 bis pH 8. Geeignet sind darüber hinaus Reverse Phase-Träger, die im Rahmen der Hochdruckflüssigkeits-Chromatographie (HPLC) eingesetzt werden. Ein weiteres Isolierungsverfahren ist die Verwendung von Molekularsieben wie Fractogel® TSK HW-40S oder Sephadex® LH-20.

Als Ausgangsstoff für die Herstellung der Thioperylenolderivate dient das mikrobiologisch hergestellte Thioperylenol. Die Herstellung der Verbindungen der Formel I erfolgt in an sich bekannter Weise, beispielsweise kann die Epoxidgruppe des Thioperylens durch Hydrolyse oder Solvolyse zu Alkoholen oder Estern umgesetzt werden. Epoxide sind sehr reaktionsfähige Verbindungen, die außer Wasser und Säuren auch andere nukleophile Reagenzien, wie Alkohole, Thiole, Amine, Grignard-Verbindungen in Gegenwart saurer oder basischer Katalysatoren addieren. Die Addition von Blausäure führt weiter zu β-Hydroxy-propionitrilderivaten. Darüber hinaus können Epoxide zu Carbonyl-Derivaten umgelagert werden, zum Beispiel durch Lewis-Säuren katalysiert. Die Reaktionsprodukte können ferner weiteren Reaktionen unterworfen werden. Diese Umsetzungen sind an sich bekannt und werden beispielsweise von Jerry March, Advanced Organic Chemistry, John Wiley & Sons, 4th Edition, 1992, beschrieben.
Weitere Derivate erhält man wenn der 3-Thio-milchsäure-Rest des Thioperylenols reduktiv oder oxidativ umgesetzt wird. Auch kann es vorteilhaft sein das Sulfid als sogenannte Abgangsgruppe zu benützen und sie durch geeignete andere Reste in literaturbekannter Weise zu ersetzten.
Um Umsetzungen selektiv durchzuführen, kann es vorteilhaft sein vor der Reaktion geeignete, in an sich bekannter Weise, Schutzgruppen einzuführen. Die Schutzgruppen werden nach der Reaktion abgespaltet und anschließend wird das Reaktionsprodukt gereinigt.

Unter pharmakologisch verträglichen Salzen der Verbindung der Formel I versteht man sowohl anorganische als auch organische Salze, wie sie in Remington's Pharmaceutical Sciences (17. Auflage, Seite 1418 (1985)) beschrieben sind. Die Herstellung physiologisch verträglicher Salze aus zur Salzbildung befähigten Verbindungen der Formel I, einschließlich deren stereoisomeren Formen, erfolgt in an sich bekannter Weise. Die Carbonsäure bildet mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin oder Triethanolamin oder auch basischen Aminosäuren, etwa Lysin, Ornithin oder Arginin, stabile Alkali-, Erdalkali- oder gegebenenfalls substituierte Ammoniumsalze. Sofern die Verbindung der Formel I basische Gruppen aufweist, lassen sich mit starken Säuren auch stabile Säureadditionssalze herstellen. Hierfür kommen sowohl anorganische als auch organische Säuren wie Chlorwasserstoff-, Bromwasserstoff-, Schwefel-, Benzolsulfon-, Phosphor-, Methansulfon-, p-Toluolsulfon-, 4-Brombenzol-sulfon-, Trifluormethylsulfon-, Cyclohexylamidosulfon-, Essig-, Oxal-, Wein-, Bernstein- und Trifluoressigsäure in Frage.

Die Erfindung betrifft auch Arzneimittel, gekennzeichnet durch einen wirksamen Gehalt an mindestens einer Verbindung der Formel I und/oder eines physiologisch verträglichen Salzes der Verbindung der Formel I und/oder eine gegebenenfalls stereoisomere Form der Verbindung der Formel I, zusammen mit einem pharmazeutisch geeigneten und physiologisch verträglichen Trägerstoff, Zusatzstoff und/oder anderen Wirk- und Hilfsstoffen.

Aufgrund der pharmakologischen Eigenschaften eignen sich die Verbindungen der Formel I zur Prophylaxe und Therapie all solcher Erkrankungen bei denen erhöhte Blutplättclienaggregationen auftreten und die durch Thrombenbildung oder Embolien verursacht werden. Dazu gehören beispielsweise Herzinfarkt (myocardial infarction), instabile Angina pectoris (unstable angina), Hirnschlag (stroke), transitorische ischämische Attacken (transitory ischaemic attacks) oder periphere arterielle Verschlußkrankheiten (peripheral vascular disease) wie intermittierendes Hinken (intermited claudication).

Die Erfindung betrifft auch die Verwendung von mindestens einer Verbindung der Formel I und/oder alle stereoisomere Formen der Verbindung der Formel I und/oder Gemische diese Formen in jedem Verhältnis, und/oder ein physiologisch verträgliches Salz der Verbindung der Formel I zur Herstellung von Arzneimitteln zur Prophylaxe und Therapie von Erkrankungen bei denen erhöhte Blutplättchenaggregationen auftreten, wobei R1, R3 und R8 unabhängig voneinander für -OH oder =O stehen,
R2 für -OH steht,
R4 und R5 unabhängig voneinander für -OH oder Wasserstoffatom stehen, oder
R4 und R5 zusammen mit den Kohlenstoffatomen an die sie jeweils gebunden sind ein Epoxid bilden,
R6, R10 und R11 unabhängig voneinander für-OH oder Wasserstoffatom stehen, oder R10 und R11 zusammen mit den Kohlenstoffatomen an die sie jeweils gebunden sind ein Epoxid bilden,
R7 für Wasserstoffatom oder den Rest -S-CH₂-CHOH-COOH steht,
und die Bindung zwischen -C₁₄-C₁₅- eine Einfachbindung oder eine Doppelbindung ist.

Die Erfindung betrifft ferner die Verwendung von mindestens einer Verbindung der Formel I zur Herstellung von Arzneimitteln zur Prophylaxe und Therapie von Erkrankungen bei denen erhöhte Blutplättchenaggregationen auftreten, wobei Thioperylenol, eine Verbindung der Formel I, worin R1, R2, R3 und R6 jeweils für -OH stehen, R4 und R5 zusammen mit den Kohlenstoffatomen an die sie jeweils gebunden sind ein Epoxid bilden, R7 für den Rest -S-CH₂-CHOH-COOH steht, R8 für =O steht,
R10 und R11 jeweils Wasserstoffatom sind, und die Bindung zwischen -C₁₄-C₁₅- eine Einfachbindung ist,
Alterperylenol, eine Verbindung der Formel I, worin R1, R2, R5 und R6 jeweils für -OH stehen, R3 und R8 jeweils für =O stehen, R4, R7, R10 und R11 jeweils für Wasserstoffatom stehen, und die Bindung zwischen -C₁₄-C₁₅- eine Doppelbindung ist, Altertoxin I, eine Verbindung der Formel I, worin R1, R2, R5 und R6 jeweils für
-OH stehen, R3 und R8 jeweils für =O stehen, R4, R7, R10 und R11 jeweils für Wasserstoffatom stehen, und die Bindung zwischen -C₁₄-C₁₅- eine Einfachbindung ist, Altertoxin II, eine Verbindung der Formel I, worin R1, R2 und R6 jeweils für -OH stehen, R3 und R8 jeweils für =O stehen, R4 und R5 zusammen mit den Kohlenstoffatomen an die sie jeweils gebunden sind ein Epoxid bilden, R7, R10 und R11 jeweils für Wasserstoffatom stehen, und die Bindung zwischen -C₁₄-C₁₅- eine Einfachbindung ist, angewendet wird.

Die Erfindung betrifft ferner die Verwendung von Altertoxin III zur Herstellung von Arzneimitteln zur Prophylaxe und Therapie von Erkrankungen bei denen erhöhte Blutplättchenaggregationen auftreten.

Geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuß- oder Sesamöl, Polyethylen-glykol und Lösungsmittel wie etwa steriles Wasser und ein- oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis von 0,1 mg/kg Körpergewicht bis 1000 mg/kg Körpergewicht, vorzugsweise von 0,2 mg/kg Körpergewicht bis 100 mg/kg Körpergewicht betragen. Sie werden zweckmäßig in Dosierungseinheiten verabreicht, die mindestens die wirksame Tagesmenge der Verbindung der Formel I, etwa bis zu 1000 mg, bevorzugt jedoch etwa 50 bis 300 mg und bei Injektionslösungen in Ampullenform bis zu etwa 300 mg, vorzugsweise aber etwa 10 bis 100 mg, enthalten.

Für die Behandlung eines erwachsenen, etwa 70 kg schweren Patienten sind je nach Wirksamkeit der Verbindung gemäß Formel I, Tagesdosen von etwa 20 mg bis 1000 mg Wirkstoff, bevorzugt etwa 100 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Ein weiterer Gegenstand der Erfindung ist der Mikroorganismus DSM 14452.

Die folgenden Beispiele sollen der näheren Erläuterung der Erfindung dienen, ohne die Breite der Erfindung in irgendeiner Weise begrenzen zu wollen.

### Beispiel 1 Herstellung einer Glycerinkultur des Pilzstammes ST 003367, DSM 14452

30 ml Nährlösung (Malzextrakt 2,0%, Hefeextrakt 0,2 %, Glucose 1,0 %, (NH₄)₂ HPO₄ 0,05 %, pH 6,0) in einem sterilen 100 ml Erlenmeyerkolben wurden mit dem Pilzstammes ST 003367, DSM 14452, beimpft und 6 Tage bei 25° C und 140 Umdrehungen pro Minute (UpM) auf einer rotierenden Schüttelmaschine inkubiert. 1,5 ml dieser Kultur wurden anschließend mit 2,5 ml 80 %igem Glycerin verdünnt und bei-135°C gelagert.

### Beispiel 2 Herstellung einer Vorkultur des Pilzes ST 003367, DSM 14452, im Erlenmeyerkolben

100 ml Nährlösung (Malzextrakt 2,0%, Hefeextrakt 0,2 %, Glucose 1,0 %, (NH₄)₂ HPO₄ 0,05 %, pH 6) in einem sterilen 300 ml Erlenmeyerkolben wurden mit dem Pilzstamm ST 003367, DSM 14452, beimpft und 4 Tage bei 25° C und 140 UpM auf einer rotierenden Schüttelmaschine inkubiert. 2 ml dieser Vorkultur wurden anschließend für die Herstellung der Hauptkulturen benötigt.

### Beispiel 3 Herstellung des Thioperylenols durch Kultur des Pilzstammes ST 3367, DSM14452.

Im Kolben:
Ein steriler 300 ml Erlenmeyerkolben mit 100 ml der folgende Nährlösung: KartoffelDextrose 2,4%, Hefeextrakt 0,2 %, pH 5,1, wurde mit einer auf einem Schrägröhrchen (gleiche Nährlösung, jedoch mit 2 % Agar) gewachsenen Kultur oder mit 2 ml einer Vorkultur, wie in Beispiel 2 beschrieben, beimpft und auf einer Schüttelmaschine bei 140 UpM und 25 °C inkubiert. Die maximale Produktion einer oder mehrerer Verbindungen der Formel I wurde nach etwa 96 Stunden erreicht.
Im Fermenter:
Die Vorkulturanzucht des Stammes ST 003367, DSM 14452, erfolgte bei 25 °C und 140 Upm im 2 L-Erlenmeyerkolben (Füllvolumen 500 mL). Nach 72 h wurde der Fermenter beimpft. Die Fermentation des Stammes erfolgte in 8 L-Fermentern. Die Bedingungen für die Fermentation wurden wie folgt eingestellt und führten zu der Produktion der erfindungsgemäßen Verbindung Thioperylenol:
Temperatur = 25°C; Begasung = 0.5 vvm; Drehzahl = 200-220 Umdrehungen pro Minute (Upm); Inokulum = 6 %; Kultivierungszeit = 96 Stunden (h).
Nährmedien:

| Vorkultur | | |
|---|---|---|
| Malzextrakt | 20 | g/L |
| Hefeextrakt | 2 | g/L |
| Glucose | 10 | g/L |
| (NH₄)₂PO₄ | 0,5 | g/l |
| | | |

| Hauptkultur | | |
|---|---|---|
| Potato Dextrose Broth | 24 | g/L |
| Hefeextrakt | 2 | g/L |
| Desmophen | 1,25 | mUL |

Durch wiederholte Zugabe von ethanolischer Polyollösung konnte die Schaumbildung unterdrückt werden. Das Produktionsmaximum wurde nach etwa 96 bis 144 Stunden erreicht.

### Beispiel 4: Isolierung des Naturstoffes Thioperylenol.

15 Liter der nach Beispiel 3 gewonnenen Kulturlösung wurden durch Zentrifugieren von der Zellmasse befreit. Das Mycel (1,5 Liter) wird mit 5 Liter Methanol extrahiert. Die klare alkoholische Phase wurde unter verminderten Druck auf etwa 2 Liter eingeengt und mit dem Kulturfitrat vereinigt. Anschließend trug man die trübe wässrige Lösung auf eine 1 L fassende, mit dem Adsorptionsharz MCI Gel® CHP20P gefüllte Säule auf. Säulenmaße: Breite x Höhe: 6 cm x 35 cm. Eluiert wurde mit einem Lösungsmittel-Gradienten von 0,1 % Ammoniumformiat-Puffer, pH 4.5 in Wasser nach Propanol-2. Der Säulenausfluß (60 mL/Minute) wurde in Fraktionen je 220 mL aufgefangen. Die Thioperylenol-haltigen Fraktionen 21 und 22, die durch HPLC-Analysen überprüft wurden, wurden gesammelt und unter verminderten Druck konzentriert. Das Thioperylenol wurde in reiner Form durch wiederholte präparative HPLC an SP 250/21 NUCLEOSIL 100-7 C18 HD^{®}-Säulen (Macherey-Nagel, Düren) mit dem Elutionsmittel 0,1 % Ammon-iumformiat, pH 4.8, in Wasser / 95 % Acetonitril im Gradienten-Verfahren gewonnen. Der Säulendurchfluß betrug 25 mL / Minute, das Thioperylenol wurde mit etwa 25 % Acetonitrilanteil von der Trennsäule eluiert. Durch langsames Konzentrieren unter verminderten Druck wurden 10 mg kristallines Thioperylenol erhalten.

### Beispiel 5: Hochdruckflüssigkeitschromatographie (HPLC) des Thioperylenols.

| | |
|---|---|
| Säule: | YMC-Pack Pro C18®, AS-303, 250 x 4.6 mm, S-5 µm; |
| Mobile Phase: | 0 bis 2 Minuten: 0,02 % Trifluoressigsäure (TFA), |
| | 2 bis 20 Minuten: 0 % bis 100 % Acetonitril in 0, 1 % TFA, |
| | 20 bis 25 Minuten: 100 % Acetonitril. |
| Flußgeschwindigkeit: | 1 mL pro Minute, |
| Detektion durch UV-Absorption bei 210 nm. | |
| Es wurde für das Thioperylenol die Retentionszeit von 12,1 Minuten gefunden. | |

### Beispiel 6: Eigenschaften des Thioperylenols.

Die physikalisch-chemischen sowie spektroskopischen Eigenschaften des Thioperylenols lassen sich wie folgt zusammenfassen:
Aussehen:

Bräunliche Kristalle, die in mittelpolaren, polaren organischen Lösungsmitteln und in wässrigen neutralen Puffern löslich sind. Stabil in neutralem und mild saurem, nicht oxidierendem Milieu, jedoch unbeständig in stark-saurer und stark-alkalischer Lösung.

| | | | |
|---|---|---|---|
| Summenformel: | C₂₃H₂₀O₉S, | Molekulargewicht: | 472,47 |
| ¹H- und ¹³C-NMR: | siehe Tabelle 1; | | |

UV-Maxima in Wasser/Acetonitril (1 zu 1), pH 3.0 : 216 nm, 259 nm, 292 nm und 383 nm.

**Tabelle 1: Chemische Verschiebungen von V-2474 in DMSO bei 300 K.**

| Position | ¹H | ¹³C |
|---|---|---|
| 1 | - | 160.14 |
| 2 | 7.01 | 118.22 |
| 3 | 8.05 | 132.57 |
| 4 | - | 125.12 |
| 5 | - | 137.09 |
| 6 | - | 113.86 |
| 7 | - | 122.82 |
| 8 | 7.53 | 123.75 |
| 9 | 6.79 | 113.50 |
| 10 | - | 156.37 |
| 11 | - | 122.66 |
| 12 | - | 128.08 |
| 13 | - | 204.10 |
| 14 | 3.62/3.06 | ~ 39.1 |
| 15 | 4.29 | 46.56 |
| 16 | - | 70.80 |
| 17 | 3.64 | 41.35 |
| 18 | 3.93 | 49.15 |
| 19 | 3.44 | 55.30 |
| 20 | 5.11 | 59.67 |
| 21 | 2.92/2.76 | 36.23 |
| 22 | 3.72 | 72.96 |
| 23 | - | 174.38 |

### Beispiel 7: Massenspektrometrische Charakterisierung des Thioperylenols.

Dem Thioperylenol wird die Masse 472 zugeordnet aufgrund folgender Befunde: ESI⁺-Spektrum lieferte schwache Peaks bei 490 amu (M+NH₄)⁺ und 962 amu (2M+NH₄)⁺. ESI⁻ Spektrum lieferte u.a. einen Peak bei 471 amu (M-H)⁻.

Mit einem FTICR-Massenspektrometer wurde im ESI- Modus unter Phosphorsäurezusatz u.a. ein Peak bei 569.0525 amu beobachtet. Der Messwert stimmt gut mit dem für (M+H₂PO₄)⁻ = C₂₃H₂₂O₁₃PS = 569.0524 amu berechneten überein (0,2 ppm Abweichung).

MS/MS-Experimente mit einem FTICR-Massenspektrometer führten zu folgenden Fragmentierungen im ESI⁻ Modus:
471 amu zu 349 amu (-C₃H₆O₃S), 331 amu (-C₃H₈O₄S), 313 amu (-C₃H₁₀O₅S), 303 amu (-C₄H₈O₅S), 285 amu (-C₄H₁₀O₆S), 261 amu (-C₆H₁₀O₆S) und kleineren Fragmenten.

### Pharmakologische Beispiele

Gemessen werden kann die Plättchenaggregations-Hemmung unter anderem durch die turbimetrische Plättchenaggregationsbestimmung nach Bom (Born, GV, Nature, 1962, 194: 927-929).

Testprinzip: Die Methode beruht auf der Eigenschaft, daß die optische Dichte einer Partikelsuspension abhängig von der Partikelzahl und nicht der Größe ist. Nach Stimulation einer Plättchensuspension setzt die Aggregation der Plättchen ein. Dies führt zum Auftreten größerer Plättchenaggregate und der Zunahme der Lichttransmission. Diese wird fortlaufend photometrisch registriert und in Kurvenform kontinuierlich aufgezeichnet. Aus den Änderungen der Lichttransmission leiten sich die Maße der Aggregabilität der Thrombozyten bzw. deren Hemmung ab.

Zur Bestimmung der Zytotoxizität, wurde die Methode der Calcein Färbung von Zellen angewandt (Hollo Z et al., Biochim Biophys Acta 11;1191(2):384-8, 1994).

Lebende Zellen inkorperieren das Substrat Calcein-Acetoxymethyl-Ester (C-AM), welches innerhalb der Zellen durch unspezifische Esterasen hydrolysiert wird. Der C-AM ist farblos und fluoresziert nicht. Innerhalb der lebenden Zelle erfolgt die Umwandlung zu dem fluoreszierenden Hydrolyseprodukt und die Vitalität der Zellen kann anhand der Fluoreszenz gemessen werden (λₑₓ:485nm / λₑₘ:538nm).

Die Tabelle 2 faßt die Hemmwirkungen einiger Hydroperylen-Derivate als IC₅₀-Wert beispielhaft zusammen. Der IC₅₀-Wert gibt die Konzentration an, die die Thrombocytenaggregation unter bestimmten Bedingungen zu 50% hemmt.

**Tabelle 2. Die Konzentrationen einiger Hydroperylen-Derivate, die die ThrombocytenAggregation zu 50% hemmen ( IC₅₀).**

| Verbindung | IC₅₀ (Plättchenaggregation) IC₅₀ (Toxizität) | |
|---|---|---|
| Thioperylenol | 7,2 µM | 61 µM |
| Alterperylenol | 1,6 µM | > 100 µM |
| Altertoxin I | 29 µM | > 100 µM |
| Altertoxin II | 2,6 µM | 10 µM |
| Altertoxin III | 6,1 µM | 20 µM |

Wie aus der Tabelle zu ersehen ist, weisen die Hydroperylenderivate neben einer Hemmung der Thrombocytenaggregation auch zum Teil eine erhebliche Zelltoxizität auf. Die Zelltoxizität ist besonders stark bei Verbindungen, die Epoxyd-Gruppen tragen. Deshalb sind von den Verbindungen der Formel I besonders diejenigen wertvoll, die zwar eine hemmende Wirkung auf die Plättchenaggregation besitzen, aber wenig toxisch sind. Bevorzugt sind also epoxydfreie Hydroperylenol-Derivate.

### Beispiel 8: Test auf Hemmung der Thrombocytenaggregation.

Für die Durchführung des Thrombocytenaggregationstestes werden folgende Reagenzien benötigt:

| Materialien | Lieferant | Katalog Nr. |
|---|---|---|
| Water tissue culture grade | Sigma | W-3500 |
| NaCl | Merck | 1.06404 |
| KCI | Merck | 1.04933 |
| CaCl₂ | Merck | 1.02083 |
| Albumin, Rind (BSA) | Sigma | A-6003 |
| Calcein AM | Molecular Probes | C-1430 |
| Collagen reagent | Nycomed | 5368 |
| Human alpha Thrombin | Haemochrom Diagnostica | HT1002A |

Der Assay wurde folgendermaßen durchgeführt:
Tyrode Puffer:

| | |
|---|---|
| NaCl | 120mM |
| KCI | 2,6mM |
| NaHCO3 | 12mM |
| NaH₂PO₄ x H₂O | 0,39mM |
| Hepes | 10mM |
| Glucose | 5,5mM |
| BSA | 0,35% |

Zugabe von Glucose und BSA täglich frisch
pH-Wert auf 7,4 einstellen
Calcein AM: 1 mg Calcein AM in 1 ml DMSO (1 mM )
Humanes α-Thrombin (Stammlösung): 1000 units in 1 ml NaCl 0,9%
Endkonzentration Aktivator: 0,05 U/ml Thrombin oder 1 µg/ml Collagen Wortmannin, Stammlösung: 1 mg in 233,43 µl DMSO (10 mM).
Aggregation in Aggregometer (PAP 4)
- Zellzahl 3 x 10⁵ Thrombozyten/µl
- Ansatz in siliconisierten Microröhrchen aus Glas
- Gesamtvolumen 400 µl/tube

pro Microröhrchen:
- 320 µl Thrombozyten
- 20µl CaCl₂ 10mM (f.c. 0,5 mM)
- 20µl Testsubstanz
- 40µl Agonist

Kontrollen:
- blank: 400 µl Puffer (Tyrode, 0,35%BSA)
- Negativ-Kontrolle: 320 µl Thrombozyten
   40µl CaCl₂ 5 mM (f.c. 0,5 mM)
   40µl Puffer
- Positv-Kontrolle: 320µl Puffer
   40µl CaCl₂ 5mM (f.c. 0,5 mM)
   40µl Aktivator

Der Leerwert für die Kanäle des Aggregometers wird zunächst mit Puffer eingestellt. Dann werden die Thrombozyten in Microtubes pipettiert und nach Zugabe der Thrombozyten im Aggregometer für ein paar Minuten auf 37°C erwärmt. Nach Zugabe eines Magnetrührers in jedes Reaktionsgefäß werden die Substanzen sowie CaCl₂ zugegeben und die Aufzeichnung des Kurvenverlaufs im Aggregometer gestartet. Nach 2 Minuten Inkubation erfolgt die Zugabe des Aktivators, bzw. von Puffer in der Kontrolle.Der Kurvenverlauf wird für weitere 6 min bei 37 °C und einer Rührergeschwindigkeit von 1050 Umdrehungen pro Minute aufgezeichnet

## Patentansprüche

1. Verbindung der Formel I 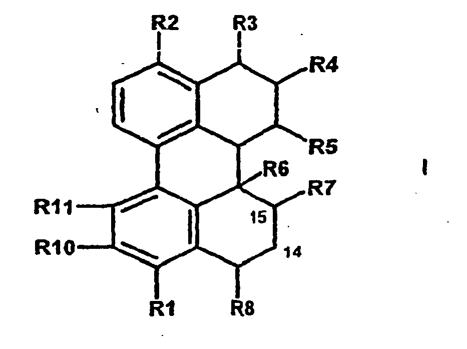 und/oder alle stereoisomere Formen der Verbindung der Formel I und/oder Gemische diese Formen in jedem Verhältnis, und/oder ein physiologisch verträgliches Salz der Verbindung der Formel I, wobei
R1, R3 und R8 unabhängig voneinander für -OH oder =O stehen,
R2 für -OH steht,
R4 und R5 unabhängig voneinander für -OH oder Wasserstoffatom stehen, oder R4 und R5 zusammen mit den Kohlenstoffatomen an die sie jeweils gebunden sind ein Epoxid bilden,
R6, R10 und R11 unabhängig voneinander für -OH oder Wasserstoffatom stehen, oder
R10 und R11 zusammen mit den Kohlenstoffatomen an die sie jeweils gebunden sind ein Epoxid bilden,
R7 für den Rest -S-CH₂-CHOH-COOH steht,
und die Bindung zwischen -C₁₄-C₁₅- eine Einfachbindung oder eine Doppelbindung ist.

2. Verbindung der Formel I gemäß Anspruch 1, wobei
R1, R2, R3 und R6 jeweils für -OH stehen,
R4 und R5 zusammen mit den Kohlenstoffatomen an die sie jeweils gebunden sind ein Epoxid bilden,
R7 für den Rest -S-CH₂-CHOH-COOH steht,
R8 für =O steht,
R10 und R11 jeweils für Wasserstoffatom stehen und
die Bindung zwischen -C₁₄-C₁₅- eine Einfachbindung ist,
und/oder physiologisch verträgliche Salze der Verbindung der Formel I.

3. Verbindung der Formel I gemäß der Ansprüche 1 oder 2, wobei die Verbindung der Formel I die Verbindung der Formel II 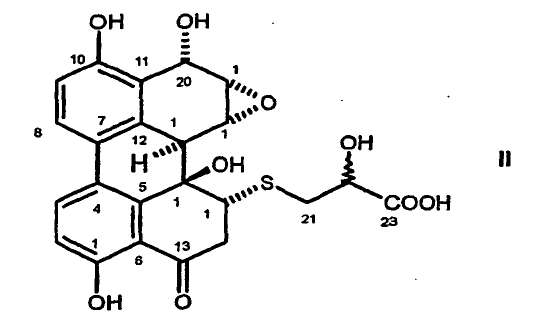 darstellt.

4. Verfahren zur Herstellung der Verbindung der Formel I gemäß einem oder mehreren der Ansprüche 1 bis 3, und/oder einer stereoisomeren Form der Verbindung der Formel und/oder Gemische diese Formen in jedem Verhältnis, und/oder ein physiologisch verträgliches Salz der Verbindung der Formel I, **dadurch gekennzeichnet, dass** man
a) den Mikroorganismus DSM 14452 oder dessen Mutanten oder Varianten in einem wäßrigen Nährmedium kultiviert und die Verbindung Thioperylenol isoliert und reinigt oder
b) Thioperylenol durch chemische Derivatisierung in eine Verbindung der Formel I überführt, oder
c) eine nach den Verfahren a) oder b) hergestellte Verbindung der Formel I, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder
d) die nach den Verfahren a), b) oder c) hergestellte Verbindung der Formel I entweder in freier Form isoliert oder im Falle des Vorliegens von sauren oder basischen Gruppen in physiologisch verträgliche Salze umwandelt.

5. Mikroorganismus DSM 14452.

6. Arzneimittel, **gekennzeichnet durch** einen wirksamen Gehalt an mindestens einer Verbindung der Formel I gemäß einem oder mehreren der Ansprüche 1 bis 3, und/oder eines physiologisch verträgliche Salzes der Verbindung der Formel I und/oder eine gegebenenfalls stereoisomere Form der Verbindung der Formel I, zusammen mit einem pharmazeutisch geeigneten und physiologisch verträglichen Trägerstoff, Zusatzstoff und/oder anderen Wirk- und Hilfsstoffen.

7. Verwendung von mindestens einer Verbindung der Formel I gemäß Anspruch 1 und/oder alle stereoisomere Formen der Verbindung der Formel I und/oder Gemische diese Formen in jedem Verhältnis, und/oder ein physiologisch verträgliches Salz der Verbindung der Formel I, zur Herstellung von Arzneimitteln zur Prophylaxe und Therapie von Erkrankungen bei denen erhöhte Blutplättchenaggregationen auftreten, wobei
R1, R3 und R8 unabhängig voneinander für -OH oder =O stehen,
R2 für -OH steht,
R4 und R5 unabhängig voneinander für -OH oder Wasserstoffatom stehen, oder R4 und R5 zusammen mit den Kohlenstoffatomen an die sie jeweils gebunden sind ein Epoxid bilden,
R6, R10 und R11 unabhängig voneinander für -OH oder Wasserstoffatom stehen, oder
R10 und R11 zusammen mit den Kohlenstoffatomen an die sie jeweils gebunden sind ein Epoxid bilden,
R7 für Wasserstoffatom oder den Rest -S-CH₂-CHOH-COOH steht,
und die Bindung zwischen -C₁₄-C₁₅- eine Einfachbindung oder eine Doppelbindung ist.

8. Verwendung gemäß Anspruch 7, wobei
Thioperylenol, eine Verbindung der Formel I, worin R1, R2, R3 und R6 jeweils für -OH stehen, R4 und R5 zusammen mit den Kohlenstoffatomen an die sie jeweils gebunden sind ein Epoxid bilden, R7 für den Rest -S-CH₂-CHOH-COOH steht,
R8 für =O steht, R10 und R11 jeweils Wasserstoffatom sind, und die Bindung zwischen -C₁₄-C₁₅- eine Einfachbindung ist,
Alterperylenol, eine Verbindung der Formel I, worin R1, R2, R5 und R6 jeweils für -OH stehen, R3 und R8 jeweils für =O stehen, R4, R7, R10 und R11 jeweils für Wasserstoffatom stehen, und die Bindung zwischen -C₁₄-C₁₅- eine Doppelbindung ist,
Altertoxin I, eine Verbindung der Formel I, worin R1, R2, R5 und R6 jeweils für -OH stehen, R3 und R8 jeweils für =O stehen, R4, R7, R10 und R11 jeweils für Wasserstoffatom stehen, und die Bindung zwischen -C₁₄-C₁₅- eine Einfachbindung ist,
Altertoxin II, eine Verbindung der Formel I, worin R1, R2 und R6 jeweils für -OH stehen, R3 und R8 jeweils für =O stehen, R4 und R5 zusammen mit den
Kohlenstoffatomen an die sie jeweils gebunden sind ein Epoxid bilden, R7, R10 und R11 jeweils für Wasserstoffatom stehen, und die Bindung zwischen -C₁₄-C₁₅- eine Einfachbindung ist,
angewendet wird.

9. Verwendung von Altertoxin III zur Herstellung von Arzneimitteln zur Prophylaxe und Therapie von Erkrankungen bei denen erhöhte Blutplättchenaggregationen auftreten.

10. Verwendung gemäß einem oder mehreren der Ansprüche 7 bis 9, **dadurch gekennzeichnet, dass** es sich bei den Erkrankungen, bei denen erhöhte Blutplättchenaggregationen auftreten, um Herzinfarkt, instabile Angina pectoris, Hirnschlag, transitorische ischämische Attacken oder periphere arterielle Verschlußkrankheiten wie intermittierendes Hinken handelt.

## Claims

1. A compound of the formula I 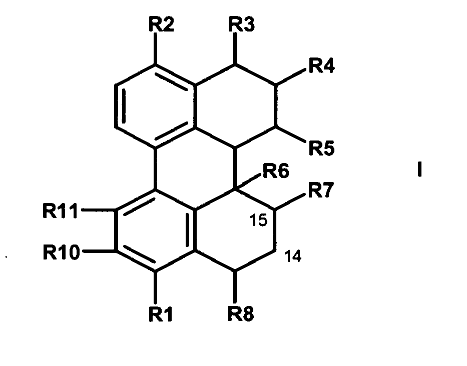 and/or all stereoisomeric forms of the compound of the formula I and/or mixtures of these forms in any ratio, and/or a physiologically tolerated salt of the compound of the formula I, where
R1, R3 and R8 are, independently of each other,
-OH or =O,
R2 is -OH,
R4 and R5 are, independently of each other -OH or a hydrogen atom, or
R4 and R5, together with the carbon atoms to which they are in each case bonded, form an epoxide,
R6, R10 and R11 are, independently of each other,
-OH or a hydrogen atom, or
R10 and R11, together with the carbon atoms to which they are in each case bonded, form an epoxide,
R7 is the radical -S-CH₂-CHOH-COOH,
and the bond between -C₁₄-C₁₅- is a single bond or a double bond.

2. A compound of the formula I as claimed in claim 1, where
R1, R2, R3 and R6 are in each case -OH,
R4 and R5, together with the carbon atoms to which they are in each case bonded, form an epoxide,
R7 is the radical -S-CH₂-CHOH-COOH,
R8 is =O,
R10 and R11 are in each case a hydrogen atom, and the bond between -C₁₄-C₁₅- is a single bond,
and/or physiologically tolerated salts of the compound of the formula I.

3. A compound of the formula I as claimed in claim 1 or 2, wherein the compound of the formula I is the compound of the formula II

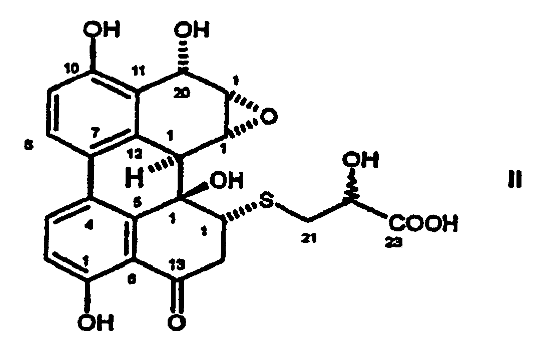

4. A process for preparing the compound of the formula I as claimed in one or more of claims 1 to 3, and/or a stereoisomeric form of the compound of the formula I and/or mixtures of these forms in any ratio, and/or a physiologically tolerated salt of the compound of the formula I, which comprises
a) culturing the microorganism DSM 14452, or its mutants or variants, in an aqueous nutrient medium and isolating and purifying the compound thioperylenol, or
b) converting thioperylenol, by means of chemical derivatization, into a compound of the formula I, or
c) resolving a compound of the formula I, which has been prepared by methods a) or b) and which, because of its chemical structure, appears in enantiomeric forms, into the pure enantiomers by forming salts with enantiomerically pure acids or bases, by chromatography on chiral stationary phases or by derivatizing with chiral, enantiomerically pure compounds such as amino acids, separating the diastereomers which are thus obtained and eliminating the chiral auxiliary groups, or
d) either isolating the compound of the formula I which has been prepared by the methods a), b) or c) in free form or, when acidic or basic groups are present, converting it into physiologically tolerated salts.

5. A microorganism DSM 14452.

6. A pharmaceutical which has an effective content of at least one compound of the formula I as claimed in one or more of claims 1 to 3, and/or of a physiologically tolerated salt of the compound of the formula I and/or of an optionally stereoisomeric form of the compound of the formula I, together with a pharmaceutically suitable and physiologically tolerated carrier substance or additive and/or other active compounds and auxiliary substances.

7. The use of at least one compound of the formula I as claimed in claim 1 and/or all stereoisomeric forms of the compound of the formula I and/or mixtures of these forms in any ratio, and/or a physiologically tolerated salt of the compound of the formula I, for producing pharmaceuticals for the prophylaxis and therapy of diseases in which high blood platelet aggregations occur, where
R1, R3 and R8 are, independently of each other, -OH or =O,
R2 is -OH,
R4 and R5 are, independently of each other -OH or a hydrogen atom, or
R4 and R5, together with the carbon atoms to which they are in each case bonded, form an epoxide,
R6, R10 and R11 are, independently of each other, -OH or a hydrogen atom, or
R10 and R11, together with the carbon atoms to which they are in each case bonded, form an epoxide,
R7 is a hydrogen atom or the radical -S-CH₂-CHOH-COOH,
and the bond between -C₁₄-C₁₅- is a single bond or a double bond.

8. The use as claimed in claim 7, where
thioperylenol, a compound of the formula I, in which R1, R2, R3 and R6 are in each case -OH, R4 and R5, together with the carbon atoms to which they are in each case bonded, form an epoxide, R7 is the radical -S-CH₂-CHOH-COOH, R8 is =O, R10 and R11 are in each case a hydrogen atom and the bond between -C₁₄-C₁₅- is a single bond,
alterperylenol, a compound of the formula I, in which R1, R2, R5 and R6 are in each case -OH, R3 and R8 are in each case =O, R4, R7, R10 and R11 are in each case a hydrogen atom, and the bond between -C₁₄-C₁₅- is a double bond,
altertoxin I, a compound of the formula I in which R1, R2, R5 and R6 are in each case -OH, R3 and R8 are in each case =O, R4, R7, R10 and R11 are in each case a hydrogen atom, and the bond between -C₁₄-C₁₅- is a single bond,
altertoxin II, a compound of the formula I, in which R1, R2 and R6 are in each case -OH, R3 and R8 are in each case =O, R4 and R5, together with the carbon atoms to which they are in each case bonded, form an epoxide, R7, R10 and R11 are in each case a hydrogen atom, and the bond between -C₁₄-C₁₅- is a single bond, is used.

9. The use of altertoxin III for producing pharmaceuticals for the prophylaxis and therapy of diseases in which high blood platelet aggregations occur.

10. The use as claimed in one or more of claims 7 to 9, wherein the diseases in which high blood platelet aggregations occur are myocardial infarction, unstable angina pectoris, stroke, transitory ischemic attacks and peripheral arterial occlusion diseases such as intermittent claudication.

## Revendications

1. Composé de formule I 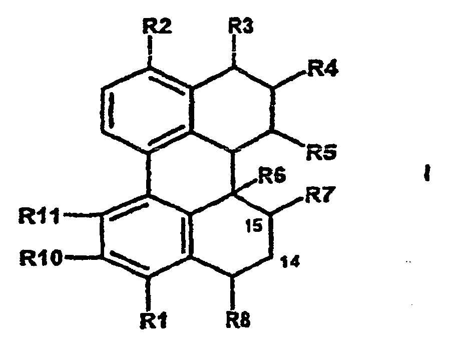 et/ou toutes les formes stéréoisomères du composé de formule I et/ou des mélanges de ces formes en un rapport quelconque, et/ou un sel physiologiquement toléré du composé de formule I, dans laquelle :
R1, R3 et R8 représentent, indépendamment les uns des autres, un groupe -OH ou un groupe =O,
R2 représente un groupe -OH,
R4 et R5 représentent, indépendamment l'un de l'autre, un groupe -OH ou un atome d'hydrogène, ou
R4 et R5, conjointement avec les atomes de carbone auxquels ils sont liés dans chaque cas, forment un époxyde,
R6, R10 et R11 représentent, indépendamment les uns des autres, un groupe -OH ou un atome d'hydrogène, ou
R10 et R11, conjointement avec les atomes de carbone auxquels ils sont liés dans chaque cas, forment un époxyde,
R7 représente le radical -S-CH₂-CHOH-COOH,
et la liaison entre -C₁₄-C₁₅- est une liaison simple ou une double liaison.

2. Composé de formule I selon la revendication 1, dans laquelle :
R1, R2, R3 et R6 représentent, dans chaque cas, un groupe -OH,
R4 et R5, conjointement avec les atomes de carbone auxquels ils sont liés dans chaque cas, forment un époxyde,
R7 représente le radical -S-CH₂-CHOH-COOH,
R8 représente un groupe =O,
R10 et R11 représentent, dans chaque cas, un atome d'hydrogène, et
la liaison entre -C₁₄-C₁₅- est une liaison simple,
et/ou des sels physiologiquement tolérés du composé de formule I.

3. Composé de formule I selon les revendications 1 ou 2, où le composé de formule I représente le composé de formule II

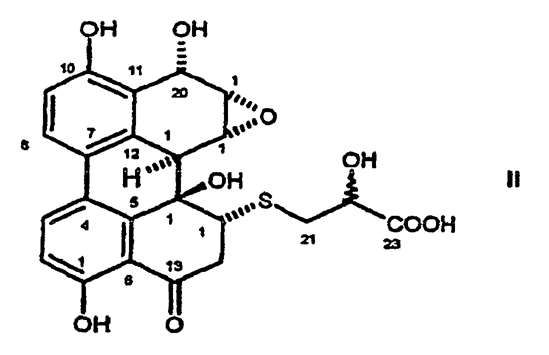

4. Procédé de préparation du composé de formule I, selon l'une ou plusieurs des revendications 1 à 3, et/ou d'une forme stéréoisomère du composé de formule I et/ou de mélanges de ces formes en un rapport quelconque, et/ou d'un sel physiologiquement toléré du composé de formule I, **caractérisé en ce que** :
a) le microorganisme DSM 14452 ou ses mutants ou variantes sont cultivés dans un milieu nutritif aqueux, et le composé thiopérylénol est isolé et purifié, ou
b) le thiopérylénol est transformé par dérivatisation chimique en un composé de formule I, ou
c) un composé de formule I préparé conformément aux procédés a) ou b), qui se présente sous des formes énantiomères en raison de sa structure chimique, est dédoublé en les énantiomères purs par formation d'un sel avec des acides ou des bases énantiomériquement purs, par chromatographie sur des phases stationnaires chirales ou par dérivatisation au moyen de composés chiraux énantiomériquement purs, tels que des acides aminés, par séparation des diastéréo-isomères ainsi obtenus et par clivage des groupes auxiliaires chiraux, ou
d) le composé de formule I, préparé conformément aux procédés a), b) ou c), est soit isolé sous la forme libre, soit transformé en des sels physiologiquement tolérés dans le cas où des groupes acides ou basiques sont présents.

5. Microorganisme DSM 14452.

6. Agent pharmaceutique, **caractérisé par** une teneur active en au moins un composé de formule I, selon l'une ou plusieurs des revendications 1 à 3, et/ou un sel physiologiquement toléré du composé de formule I et/ou une forme éventuellement stéréoisomère du composé de formule I, conjointement avec un support, un additif et/ou d'autres ingrédients actifs et substances auxiliaires, pharmaceutiquement appropriés et physiologiquement tolérés.

7. Utilisation d'au moins un composé de formule I, selon la revendication 1, et/ou de toutes les formes stéréoisomères du composé de formule I et/ou de mélanges de ces formes en un rapport quelconque, et/ou d'un sel physiologiquement toléré du composé de formule I, pour la production d'agents pharmaceutiques destinés à la prophylaxie et à la thérapie de maladies pour lesquelles il se produit des agrégations accrues de plaquettes sanguines, dans laquelle :
R1, R3 et R8 représentent, indépendamment les uns des autres, un groupe -OH ou un groupe =O, R2 représente un groupe -OH,
R4 et R5 représentent, indépendamment l'un de l'autre, un groupe -OH ou un atome d'hydrogène, ou
R4 et R5, conjointement avec les atomes de carbone auxquels ils sont liés dans chaque cas, forment un époxyde,
R6, R10 et R11 représentent, indépendamment les uns des autres, un groupe -OH ou un atome d'hydrogène, ou
R10 et R11, conjointement avec les atomes de carbone auxquels ils sont liés dans chaque cas, forment un époxyde,
R7 représente un atome d'hydrogène ou le radical -S-CH₂-CHOH-COOH,
et la liaison entre -C₁₄-C₁₅- est une liaison simple ou une double liaison.

8. Utilisation selon la revendication 7, dans laquelle on utilise :
du thiopérylénol, un composé de formule I, dans laquelle R1, R2, R3 et R6 représentent, dans chaque cas, un groupe -OH, R4 et R5 , conjointement avec les atomes de carbone auxquels ils sont liés dans chaque cas, forment un époxyde, R7 représente le radical -S-CH₂-CHOH-COOH, R8 représente un groupe =O, R10 et R11 représentent, dans chaque cas, un atome d'hydrogène et la liaison entre -C₁₄-C₁₅- est une liaison simple,
de l'alterpérylénol, un composé de formule I, dans laquelle R1, R2, R5 et R6 représentent, dans chaque cas, un groupe -OH, R3 et R8 représentent, dans chaque cas, un groupe =O, R4, R7, R10 et R11 représentent, dans chaque cas, un atome d'hydrogène, et la liaison entre -C₁₄-C₁₅- est une double liaison,
de l'altertoxine I, un composé de formule I, dans laquelle R1, R2, R5 et R6 représentent, dans chaque cas, un groupe -OH, R3 et R8 représentent, dans chaque cas, un groupe =O, R4, R7, R10 et R11 représentent, dans chaque cas, un atome d'hydrogène, et la liaison entre -C₁₄-C₁₅- est une liaison simple,
de l'altertoxine II, un composé de formule I, dans laquelle R1, R2 et R6 représentent, dans chaque cas, un groupe -OH, R3 et R8 représentent, dans chaque cas, un groupe =O, R4 et R5, conjointement avec les atomes de carbone auxquels ils sont liés dans chaque cas, forment un époxyde, R7, R10 et R11 représentent, dans chaque cas, un atome d'hydrogène, et la liaison entre -C₁₄-C₁₅- est une liaison simple.

9. Utilisation de l'altertoxine III pour la production d'agents pharmaceutiques destinés à la prophylaxie et à la thérapie de maladies pour lesquelles il se produit des agrégations accrues de plaquettes sanguines.

10. Utilisation selon l'une ou plusieurs des revendications 7 à 9, **caractérisée en ce que** les maladies, pour lesquelles il se produit des agrégations accrues de plaquettes sanguines, sont l'infarctus du myocarde, l'angine de poitrine instable, un accident vasculaire cérébral, des attaques ischémiques transitoires ou des maladies d'occlusion artérielle périphérique, telles que la claudication intermittente.
